# EUROPEAN PATENT SPECIFICATION

(11) **EP 3 827 073 B1**
(45) Date of publication and mention of the grant of the patent: **17.12.2025**
(21) Application number: 19841882.4
(22) Date of filing: 23.07.2019
(51) Int. Cl.: C12N 1/04, C12N 1/14

(54) **METHOD OF PRODUCING A MYCOLOGICAL PRODUCT AND PRODUCT MADE THEREBY**
VERFAHREN ZUR HERSTELLUNG EINES MYKOLOGISCHEN PRODUKTES UND DAMIT HERGESTELLTES PRODUKT
PROCÉDÉ DE PRODUCTION D'UN PRODUIT MYCOLOGIQUE ET PRODUIT AINSI OBTENU

(30) Priority: 23.07.2018 US 201862701906 P
(43) Date of publication of application: 02.06.2021
(73) Proprietor: Ecovative LLC, Green Island, NY 12183 (US)
(72) Inventor: CARLTON, Alex, Troy, NY 12180 (US); BAYER, Eben, Troy, NY 12182 (US); MCINTYRE, Gavin, Troy, NY 12180 (US); KAPLAN-BIE, Jessie, Troy, NY 12180 (US)
(74) Representative: Mewburn Ellis LLP
(86) International application number: PCT/US2019/042941
(87) International publication number: WO 2020/023450

(56) References cited:
- EP-B1- 2 485 779
- WO-A1-2015/024751
- WO-A1-2017/056059
- WO-A1-2017/136950
- WO-A1-2018/183735
- WO-A1-2019/099474
- WO-A1-2019/237059
- WO-A1-2020/106743
- WO-A2-2008/073489
- WO-A2-2012/122092
- WO-A2-2019/246636
- US-A- 3 885 048
- US-A- 4 036 122
- US-A- 5 979 109
- US-A1- 2009 191 289
- US-A1- 2012 076 895
- US-A1- 2013 210 327
- US-A1- 2013 263 500
- US-A1- 2015 033 620
- US-A1- 2017 071 214
- US-A1- 2018 282 529
- JAVIER ENRIONE ET AL: "Edible Scaffolds Based on Non-Mammalian Biopolymers for Myoblast Growth", MATERIALS, vol. 10, no. 12, 8 December 2017 (2017-12-08), pages 1404, XP055621815, DOI: 10.3390/ma10121404
- MUHAMMAD SAJID ARSHAD; MIRAL JAVED; MUHAMMAD SOHAIB; FARHAN SAEED; ALI IMRAN; ZAID AMJAD: "Tissue engineering approaches to develop cultured meat from cells: A mini review", COGENT FOOD AND AGRICULTURE, vol. 3, no. 1, 1320814, 20 May 2017 (2017-05-20), pages 1 - 11, XP055664493, DOI: 10.1080/23311932.2017.1320814
- NEIL STEPHENS; LUCY DI SILVIO; ILLTUD DUNSFORD; MARIANNE ELLIS; ABIGAIL GLENCROSS; ALEXANDRA SEXTON: "Bringing cultured meat to market: Technical, socio-political, and regulatory challenges in cellular agriculture", TRENDS IN FOOD SCIENCE AND TECHNOLOGY, vol. 78, 27 April 2018 (2018-04-27), pages 155 - 166, XP055665489, ISSN: 0924-2244, DOI: 10.1016/j.tifs.2018.04.010
- PATRICK J SCHANER; MARTIN NIELS D; TULENKO THOMAS N; SHAPIRO IRVING M; TAROLA NICHOLAS A; LEICHTER RHODA F; CARABASI R ANTHONY; DI: "Decellularized vein as a potential scaffold for vascular tissue engineering", JOURNAL OF VASCULAR SURGERY, vol. 40, no. 1, 1 July 2004 (2004-07-01), pages 146 - 153, XP055028034, ISSN: 0741-5214, DOI: 10.1016/j.jvs.2004.03.033
- LUIS BELARDINELLI , GERRIT ISENBERG : "Actions of Adenosine and Isoproterenol on Isolated Mammalian Ventricular Myocytes", CIRCULATION RESEARCH, vol. 53, no. 3, 1 September 1983 (1983-09-01), pages 287 - 297, XP055680967, DOI: 10.1161/01.RES.53.3.287

## Description

This invention relates to a method of producing a mycological product and the product made thereby. More particularly, this invention relates to a method of producing mushroom mycelium as a nutritious matrix. Still more particularly, this invention relates to a method of producing mushroom mycelium as a nutritious matrix for cell cultures and for foodstuffs.

### Background of the invention

As is known from US Patent 9,485,917, a self-supporting composite material may be made of a substrate of discrete particles and a network of interconnected mycelia cells extending through and around the discrete particles and bonding the discrete particles together. In general, these composite materials may be classified as mycological biocomposites comprised of lignocellulosic waste materials, fungal cellular tissue, and potentially supplemental nutrients (minerals, vitamins, and the like).

As is known from published US Patent Application 2015/0033620, a mycological biopolymer product consisting entirely of fungal mycelium may be made by inoculating a nutritive substrate with a selected fungus in a sealed environment except for a void space, which space is subsequently filled with a network of fungal mycelium. The environmental conditions for producing the mycological biopolymer product, i.e. a high carbon dioxide (CO₂) content i.e. from 5% to 7% by volume and an elevated temperature i.e. from 29°C to 35°C (85°F. to 95°F.) prevent full differentiation of the fungus into a mushroom. There are no stipe, cap, or spores produced. The biopolymer product grows into the void space of the tool, filling the space with an undifferentiated mycelium chitin-polymer, which is subsequently extracted from the substrate and dried.

As is also known from pending US Patent Application 16/190,585, filed November 14, 2018, another method of growing a biopolymer material employs incubation of a growth media comprised of nutritive substrate and a fungus in containers that are placed in a closed incubation chamber with air flows passed over each container while the chamber is maintained with a predetermined environment of humidity, temperature, carbon dioxide and oxygen. The mycological biopolymer is grown into a panel at a dry density of 0.0080 to 0.064 g/cm³ (0.5 to 4 pounds per cubic foot) on a dry mass basis.

WO 2012/122092 A2 describes a process of growing a homogeneous polymer matrix. WO 2015/024751 A1 describes a food composition comprising a layer that comprises a fungus and a water-soluble biopolymer. WO 2008073489 A2 describes a composite material comprising of a substrate of discrete particles and a network of interconnected mycelia cells. US 2013263500 A1 describes a growth medium formed as an inoculum including a preselected fungus and a nutrient material. US 3885048 A describes simulated meat, fish and dairy products prepared from a fermented vegetable product. WO 2017/136950 A1 describes a scaffold biomaterial comprising decellularized plant or fungal tissue. Javier Enrione et al. "Edible Scaffolds Based on Non-Mammalian Biopolymers for Myoblast Growth", Materials, vol. 10, no. 12, 8 December 2017, page 1404, describes scaffold materials formulated using non-mammalian sources for myoblast growth. WO 2019/237059 A1 describes a method of growing fungal mycelium and forming edible food products. WO 2018/183735 A1 describes a mycological biopolymer material treatment that enhances or retains inherent material properties. WO 2019/099474 A1 describes a method of growing a biopolymer material using incubation of a growth media. WO 2019/246636 A2 describes a mycelial foam containing macroscopic void spaces that are formed by filler elements. WO 2020/106743 A1 describes methods for generating mycelial scaffolds for use in several technologies.

It is an object of the invention to provide a mycological biopolymer material for use in making functional products.

It is another object of the invention to provide a mycological biopolymer material that can be used to create a custom, mass-produced, non-animal matrix for the production of food, for use in biomedical applications, and the like.

Briefly, the invention provides a method to create a custom, mass-produced, non-animal matrix for the production of food, for use in biomedical applications, or the like.

In a first aspect, the invention provides a method of producing a mycological product, comprising growing a porous tissue of a mycological polymer consisting entirely of fungal mycelium on a growth media comprised of nutritive substrate and a fungus while preventing full differentiation of said fungus into a mushroom, such as described in US Patent Application 16/190,585. This step occurs within a closed incubation chamber maintained with a predetermined environment of humidity, temperature, carbon dioxide and oxygen sufficient to produce a mycelium biopolymer while preventing full differentiation of said fungus into a mushroom. The method also includes removing a panel of mycological polymer from said growth media; and infusing the panel with at least one additive, wherein the additive is selected from the group consisting of plant-derived proteins, fats, micronutrients and flavoring ingredients to mimic animal-derived meat products in the panel.

In a second aspect, the invention provides a panel of mycological biopolymer consisting entirely of fungal mycelium with an additive of at least one of plant-derived proteins, fats, micronutrients and flavoring ingredients to mimic animal-derived meat products in the panel; further comprising: inoculated bovine myocytes therein; or blocking compounds for increasing shelf-life of said panel.

In one embodiment the method further comprises packaging the panel for use.

The method (process) allows for the production of large, inert, tissue panels that can be further modified to generate a material with a custom texture, flavor, and nutritional profile for use as a foodstuff or a tissue scaffold.

The method involves tailoring the density, morphology, and composition of the undifferentiated fungal material during growth and/or the use of post-processes, to improve mouth-feel and/or affinity toward flavors, fats, cellular cultures, or the like.

In one embodiment, the growth conditions in the incubation chamber are altered to yield a well-aligned macromolecular structure, resembling meat, which can then be amended with flavorings and other additives including, but not limited to, proteins, fats, flavors, aromatics, heme molecules, micronutrients, and colorants.

In a second embodiment, flavorings and other additives are deposited on the growth media during the growth process, either through liquid or solid deposition, or though natural cellular uptake (bioadsorbtion), e.g., increasing mineral content in the growth media, to increase final content in the panel of tissue.

In a third embodiment, unwanted residues (e.g., malodors, enzymes that effect shelf-stability, and the like) are removed from the panel through either post-processing, or the altering of incubation conditions.

In a fourth embodiment, the incubation and/or post-process conditions are tuned to yield a panel of tissue that, texturally, resembles animal meat (e.g., increasing alignment and decreasing growth density via temperature and airflow controls and/or mechanically, enzymatically, or chemically altering the structure of the tissue.

In a fifth embodiment, the panel of tissue (whole, or washed of any interfering residues) can be mechanically tenderized to density the native tissue (e.g., by mechanical compression, vacuum condensing, needling to entangle mycelium fibers) or to further orient fibers (e.g., calendar roller compression in the plane of fiber orientation). Additional ingredients such as a proteins, fats, flavors, aromatics, heme molecules, micronutrients, and colorants can be imparted into the mycelium matrix either before or immediately following the tenderization.

In a sixth embodiment, the panel of tissue (whole, or washed of any interfering residues) is used as a three-dimensional matrix in which non-fungal tissue cells can be supported and cultured, allowing for the in vitro production of tissue for meat consumption, or for use in biomedical applications. This tissue can be engineered, using growth conditions or post-processing, to increase the affinity for desired cell growth (e.g., increasing or decreasing porosity, increasing or decreasing mycellial diameter, deacetylation of the chitin, and the like).

These and other objects and advantages will become more apparent from the following detailed description.

The method of producing a mycological product comprises an initial step of growing a porous tissue of a mycological polymer consisting entirely of fungal mycelium on a growth media comprised of nutritive substrate and a fungus while preventing full differentiation of said fungus into a mushroom, such as described in US Patent Application 16/190,585. This step occurs within a closed incubation chamber maintained with a predetermined environment of humidity, temperature, carbon dioxide and oxygen sufficient to produce a mycelium biopolymer while preventing full differentiation of said fungus into a mushroom. Thereafter, a panel of mycological polymer is removed from the growth media; and the panel is infused with at least one additive, wherein the additive is selected from the group consisting of plant-derived proteins, fats, micronutrients and flavoring ingredients to mimic animal-derived meat products in the panel. In one embodiment the panel is removed from the growth media by slicing and then packaged for use.

The growth media may be as described in US Patent Application 16/190,585 or may be made basically of enzymatically available carbon and nitrogen sources (e.g., lignocellulosic biomass, chitinous biomass, carbohydrates) augmented with the additional micronutirents desired in the final product (e.g., minerals, vitamins).

Likewise, the fungal mycelium may be as described in US Patent Application 16/190,585 or may be made basically of an interconnected network of microscopic fibrils composed of chitin encapsulated in a matrix for beta glucans and protein.

In accordance with the method, the panel of mycological polymer is post-processed to impart desired characteristics thereto. The panel is infused with at least one additive selected form the group consisting of plant-derived proteins, fats, micronutrients and flavoring ingredients to mimic animal-derived meat products in said panel. Also, the additive may be a plant-derived additive, a cell derived additive, a fermented bacterial or fungal derived additive and an animal derived additive.

Alternatively, the growth conditions of the growth media may be tailored to obtain a desired density, morphology, and/or composition of the undifferentiated fungal material with or without the use of post-processes.

The following examples are provided to indicate the scope of the method.

### Example 1:

1. An 46x28x6cm (18-inch by 11-inch by 2.5 inch) panel of the mycological biopolymer is grown under airflow (lateral flow less than or equal to 2.8 m³ (100 cubic feet) per minute) and temperature conditions (greater than or equal to 29°C (85° Fahrenheit)) designed to create a tender (i.e. easily macerated), porous tissue on a substrate composed of 15% crude protein, 33% non-fiber carbohydrates, 28% lignin and 14% crude fat.
2. The panel is extracted from the growth media via cutting and trimmed to desired size and shape.
3. The fresh panel is then vacuum infused with plant-derived proteins, fats, micronutrients and desired flavoring ingredients (e.g., bacon flavoring) to mimic animal-derived meat products.
4. The product is then vacuum packaged (with or without blanching) in sterile liquid and refrigerated until ready for consumption.

### Example 2:

1. An 46x28x6cm (18-inch by 11-inch by 2.5 inch) panel of the mycological biopolymer is grown under airflow and temperature conditions, e.g. as above, designed to create a tender, porous tissue on a substrate composed of 15% crude protein, 33% non-fiber carbohydrates, 28% lignin, 14% crude fat, and elevated levels of essential dietary minerals.
2. During growth these elevated levels of minerals will be taken up by the fungus, bioaccumulating in the mycelial tissue, rendering the final product more nutritious and balanced.
3. The panel is extracted from the growth media via cutting and trimmed to desired size and shape.
4. The panel can then be further amended with desired additives via soaking or vacuum infusion
5. The panel can then be packaged, refrigerated, and consumed.

### Example 3:

1. An 46x28x6cm (18-inch by 11-inch by 2.5 inch) panel of the mycological biopolymer is grown under airflow and temperature conditions, e.g. as above, designed to create a tender, porous tissue on a substrate composed of 15% crude protein, 33% non-fiber carbohydrates, 28% lignin and 14% crude fat.
2. During growth, desired nutrients, flavors, or other additives can be aerosolized into the growth chamber, condensing on the propagating tissue, and being incorporated into the matrix.
3. The panel is extracted from the growth media via cutting and trimmed to desired size and shape and packaged ready for consumption or cell culture.

### Example 4:

1. An 46x28x6cm (18-inch by 11-inch by 2.5 inch) panel of the mycological biopolymer is grown under airflow and temperature conditions, e.g. as above, designed to create a tender, porous tissue on a substrate composed of 15% crude protein, 33% non-fiber carbohydrates, 28% lignin and 14% crude fat, with the addition of binding compounds, e.g. ligans and chelators, that target enzymes known to reduce shelf-life and resultant odors. These binding compounds act as blocking compounds that serve to increase shelf-life.
2. After panel extraction, the panel is washed in dilute hydrogen peroxide (3.5%) and dried under vacuum at 110C and 933Pa (7torr) to remove known malodors (e.g., 2,4,6-Trichloroanisole)

### Example 5:

1. An 46x28x6cm (18-inch by 11-inch by 2.5 inch) panel of the mycological biopolymer is grown under airflow and temperature conditions, e.g. as above, designed to create a tender, porous tissue on a substrate composed of 15% crude protein, 33% non-fiber carbohydrates, 28% lignin and 14% crude fat.
2. After panel extraction, the panel is mechanically tenderized with an array of pins and then subjected to a chitinase bath to further tenderize the tissue.
3. The panel is then packaged and is ready for consumption or cell culture.

### Example 6:

1. An 46x28x6cm (18-inch by 11-inch by 2.5 inch) panel of the mycological biopolymer is grown under airflow (greater than or equal to 4.25m³ (150 cubic feet) per hour) and temperature conditions (less than or equal to 29°C (85° Fahrenheit)) designed to create a dense
   (i.e. tough, gristle-like), porous tissue on a substrate composed of 15% crude protein, 33% non-fiber carbohydrates, 28% lignin and 14% crude fat.
2. The panel is decellularized in a heated SDS bath with sonication.
3. The decellularized panel is then sterilized, and inoculated with bovine myocytes in a bath of fetal bovine serum.
4. Cells are allowed to proliferate along the "scaffold" formed by the inoculated panel, until a complete three-dimensional cellular structure is formed in vitro. The "scaffold" in this instance is a matrix of interconnected mycelium fibrils between 1 and 10 microns in diameter and a porosity of no less than 75%. The fibers that compose the matrix serve as a structure for mammalian cells to adhere to, grow along, and differentiate from. The "scaffold" is the structure that mammalian and other cells are seeded onto and around to generate differentiated tissue structures.
5. This tissue is then useful in biomedical applications or for culinary purposes. For example, Other tissue engineering scaffolds include collagen and polylactic acid fibrils. Such scaffolds, as has been demonstrated with mycelium, can be seeded with osteoblasts (bone cells), allowed to grow on the mycelium under the right media and incubation conditions, and then differentiated into osteocytes that can be calcified to create bone tissue. The same is true for culinary approaches, but in this instance myocytes, or animal muscle cells (avian, bovine) are permitted to grow on and around the mycelium matrix. Incubated in grow media (fetal bovine serum was cited), and incubation conditions (typically the body temperature of the animal in question)

The method provides a panel of mycological biopolymer consisting entirely of fungal mycelium with an additive of at least one of plant-derived proteins, fats, micronutrients and flavoring ingredients to mimic animal-derived meat products in the panel; further comprising inoculated bovine myocytes therein; or compounds to block enzymes that would reduce shelf-life. (see Example 4).

The invention thus provides a mycological biopolymer material for use in making functional products. In particular, the invention provides a mycological biopolymer material that can be used to create a custom, mass-produced, non-animal matrix for the production of food, biomedical applications, and the like.

## Claims

1. A method of producing a mycological product, comprising:
growing a porous tissue of a mycological polymer consisting entirely of fungal mycelium on a growth media comprised of nutritive substrate and a fungus while preventing full differentiation of said fungus into a mushroom;
removing a panel of mycological polymer from said growth media; and
infusing said panel with at least one additive,
wherein said additive is selected from the group consisting of plant-derived proteins, fats, micronutrients and flavoring ingredients to mimic animal-derived meat products in said panel.

2. The method of claim 1, further comprising packaging said panel for use.

3. The method of claim 1 or 2, wherein said step of infusing is performed under vacuum.

4. The method of claim 1 or 2, wherein said step of infusing comprises soaking said panel with said at least one additive.

5. The method of claim 1 or 2, wherein said at least one additive is one of a plant-derived additive, a cell derived additive, a fermented bacterial or fungal derived additive and an animal derived additive.

6. The method of claim 1 or 2, further comprising:
including elevated levels of essential dietary minerals in said growth media for bioaccumulating in said panel.

7. The method of claim 3, further comprising:
adding the flavoring ingredients to said panel by soaking or vacuum infusion.

8. The method of claim 1 or 2, further comprising:
adding blocking compounds to said growth media to increase shelf-life of said panel.

9. The method of claim 5, further comprising:
washing said panel in dilute hydrogen peroxide (3.5%); and
drying under vacuum at a temperature and pressure to remove known malodors.

10. The method of claim 1 or 2, further comprising:
mechanically tenderizing said panel, optionally wherein said tenderizing includes passing an array of pins into said panel.

11. The method of claim 10, further comprising:
thereafter placing said panel in a chitinase bath to further tenderize said panel.

12. The method of claim 1 or 2, further comprising:
decellularizing said panel in a heated sodium dodecyl sulfate (SDS) bath with sonication;
thereafter sterilizing said decellularized panel; and
inoculating said sterilized panel with bovine myocytes in a bath of fetal bovine serum to form a complete three-dimensional cellular structure in vitro.

13. A panel of mycological biopolymer consisting entirely of fungal mycelium with an additive of at least one of plant-derived proteins, fats, micronutrients and flavoring ingredients to mimic animal-derived meat products in said panel; further comprising:
inoculated bovine myocytes therein; or
blocking compounds for increasing shelf-life of said panel.

14. The panel of claim 13, further comprising one or more of colorants, aromatics, heme molecules, and minerals.

15. The panel of claim 13, wherein the panel comprises an array of pin holes.

## Patentansprüche

1. Verfahren zur Herstellung eines mykologischen Produkts, das Folgendes umfasst:
Züchten eines porösen Gewebes eines mykologischen Polymers, das vollständig aus Pilzmyzel besteht, auf einem Wachstumsmedium, das aus einem Nährstoffsubstrat und einem Pilz besteht, während eine vollständige Differenzierung des Pilzes zu einem Fruchtkörper verhindert wird;
Entfernen einer Platte aus einem mykologischen Polymer vom Wachstumsmedium; und
Infundieren der Platte mit zumindest einem Additiv,
wobei das Additiv aus der aus von Pflanzen stammenden Proteinen, Fetten, Mikronährstoffen und Geschmacksbestandteilen zur Nachahmung von von Tieren stammenden Fleischprodukten in der Platte bestehenden Gruppe ausgewählt ist.

2. Verfahren nach Anspruch 1, das weiters das Verpacken der Platte zur Verwendung umfasst.

3. Verfahren nach Anspruch 1 oder 2, wobei der Schritt des Infundierens im Vakuum durchgeführt wird.

4. Verfahren nach Anspruch 1 oder 2, wobei der Schritt des Infundierens das Einwiechen der Platte mit dem zumindest einen Additiv umfasst.

5. Verfahren nach Anspruch 1 oder 2, wobei das zumindest eine Additiv eines aus einem von einer Pflanze stammenden Additiv, einem von einer Zelle stammenden Additiv, einem von fermentierten Bakterien oder Pilzen stammenden Additiv und einem von einem Tier stammenden Additiv ist.

6. Verfahren nach Anspruch 1 oder 2, das weiters Folgendes umfasst:
Inkludieren von erhöhten Mengen von essentiellen Nährstoff-Mineralien in dem Wachstumsmedium zu ihrer Bioakkumulierung in der Platte.

7. Verfahren nach Anspruch 3, das weiters Folgendes umfasst:
Zugeben der Geschmacksbestandteile zur Platte durch Einweichen oder Vakuuminfusion.

8. Verfahren nach Anspruch 1 oder 2, das weiters Folgendes umfasst:
Zugeben von Blockierungsverbindungen zu dem Wachstumsmedium, um die Haltbarkeit der Platte zu verlängern.

9. Verfahren nach Anspruch 5, das weiters Folgendes umfasst:
Waschen der Platte in verdünntem Wasserstoffperoxid (3,5 %); und
Trocknen im Vakuum bei einer Temperatur und bei einem Druck zur Entfernung von bekannten schlechten Gerüchen.

10. Verfahren nach Anspruch 1 oder 2, das weiters Folgendes umfasst:
mechanisches Zartmachen der Platte, wobei das Zartmachen das Einführen einer Anordnung von Nadeln in die Platte umfasst.

11. Verfahren nach Anspruch 10, das weiters Folgendes umfasst:
anschließend Platzieren der Platte in einem Chitinase-Bad, um die Platte weiter zart zu machen.

12. Verfahren nach Anspruch 1 oder 2, das weiters Folgendes umfasst:
Dezellularisierung der Platte in einem erhitzten Natriumdodecylsulfat- (SDS-) Bad unter Beschallung;
danach Sterilisieren der dezellularisierten Platte; und
Inokulieren der sterilisierten Platte mit Rindermyozyten in einem Bad aus fötalem Rinderserum, um eine vollständige dreidimensionale Zellstruktur in vitro zu bilden.

13. Platte aus mykologischem Biopolymer, die vollständig aus Pilzmyzel mit einem Additiv aus zumindest einem aus von Pflanzen stammenden Proteinen, Fetten, Mikronährstoffen und Geschmacksbestandteilen zur Nachahmung von von Tieren stammenden Fleischprodukten in der Platte besteht; und die weiters Folgendes umfasst:
inokulierte Rindermyozyten darin; oder
Blockierungsverbindungen zur Verlängerung der Haltbarkeit der Platte.

14. Platte nach Anspruch 13, die weiters eines oder mehrere aus Farbstoffen, Aromastoffen, Häm-Molekülen und Mineralien umfasst.

15. Platte nach Anspruch 13, wobei die Platte eine Anordnung von Nadellöchern umfasst.

## Revendications

1. Procédé de production d'un produit mycologique, comprenant les étapes consistant à :
faire croître un tissu poreux d'un polymère mycologique constitué entièrement de mycélium fongique sur un milieu de croissance composé d'un substrat nutritif et d'une moisissure tout en empêchant une différenciation complète de ladite moisissure en un champignon ;
retirer un panneau de polymère mycologique à partir dudit milieu de croissance ; et
infuser ledit panneau avec au moins un additif,
dans lequel ledit additif est choisi dans le groupe comprenant des protéines d'origine végétale, des graisses, des micronutriment et des ingrédients aromatisants pour imiter des produits carnés d'origine animale dans ledit panneau.

2. Procédé selon la revendication 1, comprenant en outre le conditionnement dudit panneau pour utilisation.

3. Procédé selon la revendication 1 ou 2, dans lequel ladite étape d'infusion est réalisée sous vide.

4. Procédé selon la revendication 1 ou 2, dans lequel ladite étape d'infusion comprend le trempage dudit panneau avec ledit au moins un additif.

5. Procédé selon la revendication 1 ou 2, dans lequel ledit au moins un additif est un certain parmi un additif d'origine végétale, un additif d'origine cellulaire, un additif d'origine bactérienne ou fongique fermenté et un additif d'origine animale.

6. Procédé selon la revendication 1 ou 2, comprenant en outre l'étape consistant à :
inclure des niveaux élevés de minéraux alimentaires essentiels dans ledit milieu de croissance pour une bioaccumulation dans ledit panneau.

7. Procédé selon la revendication 3, comprenant en outre l'étape consistant à :
ajouter les ingrédients aromatisants audit panneau par trempage ou infusion sous vide.

8. Procédé selon la revendication 1 ou 2, comprenant en outre l'étape consistant à :
ajouter des composés bloquants auxdits milieux de croissance pour augmenter la durée de conservation dudit panneau.

9. Procédé selon la revendication 5, comprenant en outre l'étape consistant à :
laver ledit panneau dans du peroxyde d'hydrogène dilué (3,5 %) ; et
sécher sous vide à une température et une pression permettant d'éliminer des mauvaises odeurs connues.

10. Procédé selon la revendication 1 ou 2, comprenant en outre l'étape consistant à :
attendrir mécaniquement ledit panneau, facultativement dans lequel ledit attendrissement inclut l'étape consistant à faire passer un réseau de broches dans ledit panneau.

11. Procédé selon la revendication 10, comprenant en outre l'étape consistant à :
placer ensuite ledit panneau dans un bain de chitinase pour attendrir davantage ledit panneau.

12. Procédé selon la revendication 1 ou 2, comprenant en outre les étapes consistant à :
décellulariser ledit panneau dans un bain chauffé de dodécylsulfate de sodium (SDS) avec sonication ;
stériliser ensuite ledit panneau décellularisé ; et
inoculer ledit panneau stérilisé avec des myocytes bovins dans un bain de sérum bovin fœtal pour former une structure cellulaire tridimensionnelle complète in vitro.

13. Panneau de biopolymère mycologique constitué entièrement de mycélium fongique avec un additif d'au moins un parmi des protéines d'origine végétale, des graisses, des micronutriments et des ingrédients aromatisants pour imiter des produits carnés d'origine animale dans ledit panneau ; comprenant en outre :
des myocytes bovins inoculés en son sein ; ou
des composés bloquants pour augmenter la durée de conservation dudit panneau.

14. Panneau selon la revendication 13, comprenant en outre un ou plusieurs parmi des colorants aromatiques, des molécules d'hème et des minéraux.

15. Panneau selon la revendication 13, dans lequel le panneau comprend un réseau de trous de broche.
